Europäisches Patentamt

European Patent Office

Office européen des brevets

(19)

(11) Numéro de publication : **0 339 012 B1**

(12)

# FASCICULE DE BREVET EUROPEEN

(45) Date de publication du fascicule du brevet :
**10.06.92 Bulletin 92/24**

(51) Int. Cl.⁵ : **C07D 315/00**, B01J 23/89, B01J 21/08

(21) Numéro de dépôt : **89870055.4**

(22) Date de dépôt : **20.04.89**

(54) Procédé de fabrication de gamma-butyrolactone.

(30) Priorité : **22.04.88 GB 8809587**

(43) Date de publication de la demande :
**25.10.89 Bulletin 89/43**

(45) Mention de la délivrance du brevet :
**10.06.92 Bulletin 92/24**

(84) Etats contractants désignés :
**AT BE CH DE ES FR GB GR IT LI LU NL SE**

(56) Documents cités :
EP-A- 0 159 578
EP-A- 0 246 031
US-A- 3 948 805
US-A- 3 994 928
US-A- 4 087 476
US-A- 4 620 017

(73) Titulaire : **U C B, S.A.**
**326, Avenue Louise, Bte 7**
**B-1050 Bruxelles (BE)**

(72) Inventeur : **Dallons, Jean-Luc**
**4, Rozenstraat**
**B-1600 Sint-Pieters-Leeuw (BE)**
Inventeur : **Jacobs, Pierre**
**104, Strijlandstraat**
**B-1686 Gooik (BE)**
Inventeur : **Martens, Johan**
**72, Mohrfeldstraat**
**B-1090 Bruxelles (BE)**
Inventeur : **Tastenhoye, Paul**
**63, Eizerstraat**
**B-1982 Tervuren (BE)**
Inventeur : **Vanden Eynde, Ivan**
**15, Kruisheide**
**B-2850 Keerbergen (BE)**
Inventeur : **Van Gysel, August**
**97, Molenbergstraat**
**B-1710 Dilbeek (BE)**

(74) Mandataire : **Dusseldorp, Raymond et al**
**U.C.B. S.A. Département D.T.B. 326, avenue**
**Louise, Bte 7**
**B-1050 Bruxelles (BE)**

EP 0 339 012 B1

Jouve, 18, rue Saint-Denis, 75001 PARIS

## Description

La présente invention se rapporte à un nouveau procédé de fabrication de gamma-butyrolactone par hydrogénation catalytique de l'anhydride maléique en phase liquide.

Il est bien connu que l'hydrogénation catalytique de l'anhydride maléique conduit à des produits divers tels que l'anhydride succinique, la gamma-butyrolactone, le 1,4-butanediol et/ou le tétrahydrofuranne ainsi qu'à d'autres produits d'hydrogénation de moindre valeur tels que du propanol, du butanol, de l'acide butyrique, etc. et que de nombreuses tentatives ont été faites pour obtenir préférentiellement un seul de ces produits d'hydrogénation grâce à l'emploi de catalyseurs spécifiques.

Dans le but d'obtenir des rendements optima en gamma-butyrolactone, il a été proposé de synthétiser ce produit en deux stades en procédant d'abord à la transformation de l'anhydride maléique en anhydride succinique à des températures et des pressions relativement basses, puis à la transformation de l'anhydride succinique en gamma-butyrolactone à des pressions et des températures plus élevées (voir par exemple le brevet américain 4.025.534).

Cependant, le procédé en deux stades présente peu d'intérêt technique et économique pour diverses raisons.

En effet, il est nécessaire d'opérer soit dans un seul réacteur, mais en prévoyant des zones de température différentes en fonction de l'état d'avancement de la réaction, soit dans deux réacteurs opérant dans des conditions opératoires différentes. Dans ce dernier cas, il faudra en outre prévoir un moyen pour transporter le mélange réactionnel à température élevée et sous pression du réacteur de synthèse de l'anhydride succinique au réacteur de synthèse de la gamma-butyrolactone.

On comprendra donc qu'il est beaucoup plus rentable de pouvoir convertir directement l'anhydride maléique en gamma-butyrolactone en un seul stade. Toutefois, cette façon de procéder se heurte à un nombre considérable de difficultés.

En premier lieu, les réactions d'hydrogénation successives ont chacune leur propre cinétique et leurs propres conditions réactionnelles. Ainsi, l'hydrogénation de l'anhydride succinique en gamma-butyrolactone exige des conditions opératoires beaucoup plus sévères (températures et pressions considérablement plus élevées et quantité plus grande de catalyseur) que l'hydrogénation de l'anhydride maléique en anhydride succinique. Pour la conversion directe de l'anhydride maléique en gamma-butyrolactone, la quantité requise de catalyseur devra donc être adaptée à la réaction d'hydrogénation de l'anhydride succinique en gamma-butyrolactone. Mais, si l'on opère en présence d'une telle quantité de catalyseur, il se dégagera une quantité importante de chaleur en un très court laps de temps, parce que la réaction d'hydrogénation de l'anhydride maléique en anhydride succinique présente un caractère exothermique marqué. En effet, cette réaction dégage environ 32,3 kcal par mole d'anhydride succinique formé. Il faut donc tout d'abord que le catalyseur que l'on envisage d'employer pour la conversion directe de l'anhydride maléique en gamma-butyrolactone soit capable de résister à cette exothermicité pour qu'il ait une durée de vie convenable, sans quoi la rentabilité du procédé s'en trouverait sérieusement affectée.

En second lieu, il est bien connu que la gamma-butyrolactone n'est qu'un produit intermédiaire de la réaction d'hydrogénation et qu'elle peut à son tour s'hydrogéner en 1,4-butanediol et/ou en tétrahydrofuranne. Or, il faut éviter la réaction consécutive vers le 1,4-butanediol vu que ce diol réagit avec l'anhydride succinique non transformé pour former des esters sans valeur, quinécessitent une méthode de séparation élaborée et constituent en outre un poison potentiel du catalyseur. On sait en outre que la réaction d'hydrogénation peut conduire à un certain nombre de sous-produits de moindre valeur tels que du propanol, du butanol, de l'acide propionique, de l'acide butyrique, etc.

Il est donc indispensable que le catalyseur utilisé soit très sélectif pour la production de gamma-butyrolactone, minimisant ainsi la formation de ces sous-produits indésirables.

On notera que pour arrêter le processus de conversion au produit souhaité, en l'occurrence la gamma-butyrolactone, on pourrait opérer l'hydrogénation avec un faible taux de conversion. Cependant, cette façon d'opérer causerait des problèmes technologiques ardus vu la faible solubilité de l'anhydride succinique et son point de fusion élevé (119,6°C). De plus, à faible taux de conversion, il faudrait obligatoirement opérer de nombreux recyclages, ce qui serait préjudiciable à la productivité du procédé et nécessiterait en outre l'usage de conduites chauffées augmentant de manière sensible le cot de fabrication. Il faut donc que le catalyseur utilisé soit non seulement très sélectif, mais qu'il soit simultanément très actif afin de réaliser des taux de conversion très élevés dépassant si possible 90%.

Enfin, on sait que la formation de sous-produits tels que l'acide propionique et l'acide butyrique augmente lorsque l'hydrogénation est effectuée à des températures relativement élevées. C'est pourquoi, il est souhaitable que le catalyseur soit actif à des températures relativement basses afin de minimiser la formation de ces sous-produits.

2

Dans le même ordre d'idées, il est souhaitable que l'hydrogénation puisse être exécutée à des pressions relativement basses pour minimiser les frais d'installation et d'exploitation (appareillages, compresseurs, etc.).

En conclusion, la difficulté est de trouver un catalyseur qui autorise une production rentable de gamma-butyrolactone au départ de l'anhydride maléique.

Un catalyseur idéal devrait en effet satisfaire aux exigences suivantes:

(1) être capable de convertir directement l'anhydride maléique en gamma-butyrolactone en un seul stade;

(2) être très sélectif pour la production de gamma-butyrolactone (93 moles % et plus), minimisant ainsi la formation de sous-produits;

(3) être suffisamment actif pour pouvoir conduire la réaction à des conversions très élevées, de préférence supérieures à 90 moles %;

(4) être actif à des températures relativement basses (par exemple dans l'intervalle de 200 à 240°C) et à des pressions relativement basses (par exemple à des pressions inférieures à 100 bars);

(5) posséder une longue durée de vie ou de service (donc sans désactivation appréciable);

(6) avoir un prix de revient acceptable.

Lorsqu'on examine l'état de la technique dans ce domaine, on constate que la plupart des catalyseurs connus ne donnent pas entière satisfaction à la fois à toutes les exigences citées plus haut. C'est ainsi que dans le brevet belge 818.434, on propose de convertir l'anhydride maléique en gamma-butyrolactone en un seul stade, en présence d'un catalyseur qui possède une bonne sélectivité pour la production de la gamma-butyrolactone (93 à 96 moles %).

Toutefois, la composition catalytique utilisée pour atteindre ce résultat est très complexe. Elle se compose en effet de deux catalyseurs distincts comportant quatre éléments catalytiquement actifs, le premier catalyseur étant à base de nickel et de chromite de cuivre déposés sur de l'oxyde d'aluminium et du kieselguhr et le second étant à base de palladium sur du charbon actif. En outre, d'après les exemples de mise en oeuvre, l'hydrogénation de l'anhydride maléique est opérée sous des pressions relativement élevées pouvant varier de 100 à 150 bars. Dans le brevet américain 4.096.156, on signale également des sélectivités très élevées dans la conversion de l'anhydride maléique en gamma-butyrolactone (90 moles % et plus), mais ces sélectivités sont obtenues grâce à l'emploi de catalyseurs très onéreux renfermant des quantités considérables de plusieurs métaux nobles. D'après les exemples, ces catalyseurs contiennent environ 11,8% en poids de palladium (ou 5,7% en poids de palladium plus environ 9,8% en poids de platine) et 2,5 à 5,2% en poids d'argent et/ou d'or, sous la forme métallique. En outre, l'hydrogénation de l'anhydride maléique est opérée sous des pressions très élevées (189 à 215 bars). Dans le brevet américain 3.994.928, on propose un catalyseur qui contient de l'oxyde de cobalt et du palladium déposés sur du kieselguhr ou des pastilles de $SiO_2$ d'un diamètre de 2-3 mm. Le prix de revient de ce catalyseur est plus acceptable, mais d'après les exemples de mise en oeuvre, la conversion de l'anhydride maléique en gamma-butyrolactone est conduite à une température de 250°C et sous des pressions de 150 atmosphères; dès lors, si ce procédé devait être appliqué à l'échelle industrielle, il exigerait une ingénierie très élaborée et partant très onéreuse.

Dans différents autres brevets, on propose également des catalyseurs pour l'hydrogénation catalytique de l'anhydride succinique en gamma-butyrolactone (demande japonaise 33030/71; brevet américain 4.620.017). Toutefois, dans ces procédés, l'anhydride maléique doit être converti au préalable en anhydride succinique dans une unité de production séparée dans laquelle on utilise, le cas échéant, un catalyseur distinct.

Comme il a déjà été expliqué plus haut, il est beaucoup plus intéressant de disposer d'un procédé et d'un catalyseur permettant de réaliser la conversion directe de l'anhydride maléique en gamma-butyrolactone en un seul stade.

Dans la demande de brevet japonaise 33030/71, on décrit la préparation de catalyseurs bimétalliques composés de nickel et d'un métal précieux choisi parmi le palladium, le ruthénium, le platine et l'osmium. Ces catalyseurs peuvent être utilisés en l'absence d'une matière de support, mais la préférence est donnée à des catalyseurs supportés. Comme matière de support, on cite du silicagel, de l'alumine, de la silice-alumine, mais on utilise de préférence de la terre à diatomées

Dans l'unique exemple de cette demande de brevet, les catalyseurs préparés sont utilisés dans l'hydrogénation catalytique de l'anhydride succinique à une température de 260°C et sous une pression de 120 kg/cm². Le meilleur résultat est obtenu avec un catalyseur composé de nickel et de palladium. Toutefois, la sélectivité pour la production de la gamma-butyrolactone reste assez faible; elle n'atteint guère 70 moles % et il se forme en outre une quantité non négligeable de sous-produits (6,6 moles % de tétrahydrofuranne, 2,8 moles % de n-propanol et 24,1 moles % d'autres composés non identifiés) tout ceci en dépit du fait que le catalyseur utilisé contient plus de 50% en poids de métal (nickel et métal precieux) par rapport au poids total du catalyseur (métaux et matière de support).

Conformément à la présente invention, on a fait la découverte surprenante qu'en utilisant aussi du nickel en association avec du palladium comme éléments catalytiquement actifs du catalyseur, mais en choisissant toutefois comme matière de support une silice présentant une surface spécifique très élevée, il est possible de confectionner un catalyseur excellent qui satisfait à toutes les exigences technologiques et économiques rappelées plus haut aux points (1) à (6) et qui peut donc être utilise très avantageusement pour la production de la gamma-butyrolactone à partir de l'anhydride maléique en un seul stade.

Par conséquent, conformément à la présente invention, on apporte un nouveau procédé de fabrication de gamma-butyrolactone par hydrogénation catalytique de l'anhydride maléique en phase liquide avec de l'hydrogène à température élevée et sous pression en présence d'un catalyseur constitué essentiellement de nickel et de palladium déposés sur un support qui est caractérisé en ce que le support est un support à base de silice présentant une surface spécifique d'au moins 100 m$^2$/g.

Contrairement aux catalyseurs utilises dans la demande japonaise 33030/71, le catalyseur nickel-palladium utilisé dans le procédé conforme à la présente invention est déposé sur un support à base de silice présentant une surface spécifique très élevée, dont la valeur peut aller d'environ 100 m$^2$/g jusqu'à 800 m$^2$/g et davantage.

On a trouve en effet que le catalyseur nickel-palladium devient très actif lorsqu'il est déposé sur un support à base de silice à haute surface spécifique, ce qui se traduit par l'obtention de taux de conversion très élevés, de l'ordre de 90 à 98 moles % avec, en outre, d'excellentes sélectivités pour la production de gamma-butyrolactone de 95 à 97 moles % (contre un maximum de 69,5 moles % de butyrolactone formée dans la demande japonaise 33030/71). On a trouvé, en outre, que la température et la pression auxquelles le catalyseur est actif pour l'hydrogénation de l'anhydride maléique sont plus faibles et que l'on peut obtenir des résultats très favorables en opérant à une température comprise entre 200 et 240°C et sous une pression comprise dans l'intervalle de 50 à 100 bars.

Au surplus, on a observé que des résultats remarquables peuvent être obtenus avec des catalyseurs dont la teneur en nickel (sous forme de métal) est seulement de 8 à 25% en poids et dont la teneur en palladium (sous forme de métal) est seulement de 0,5 à 4% en poids par rapport au poids total du catalyseur supporté. La quantité de métal (nickel et métal précieux) requise pour la préparation des catalyseurs nickel-palladium utilisés conformément à l'invention est donc nettement plus faible comparée à celle requise pour les catalyseurs décrits dans la demande japonaise 33030/71, ce qui représente un avantage économique évident.

Il est tout à fait surprenant que l'on puisse obtenir un accroissement considérable de l'activité du catalyseur nickel-palladium grâce à l'emploi d'un support à base de silice à haute surface spécifique, car dans bon nombre de brevets relatifs à l'hydrogénation de l'anhydride maléique, la préférence est donnée à la terre à diatomées (demande japonaise 3030/71) ou à des supports similaires comme le kieselguhr, ayant une surface spécifique faible de l'ordre de 0,1 à 10 m$^2$/g (cf. brevet américain 4.096.156, colonne 3, lignes 19 à 21).

Plus récemment, dans le brevet américain 4.620.017, on a également proposé comme matière de support une silice présentant une surface spécifique d'au moins 50 m$^2$/g. Dans ce brevet, on montre que l'incorporation de zirconium ou de cérium dans un catalyseur à base de nickel, déposé sur un support de silice ayant une surface spécifique de 300 m$^2$/g, a un effet bénéfique notable sur la sélectivité pour la gamma-butyrolactone.

Toutefois, ce brevet n'enseigne nullement le rôle bénéfique de la haute surface spécifique du support de silice sur l'activité du catalyseur, découverte sur laquelle repose principalement le procédé conforme à l'invention. Dans ce brevet, la silice à haute surface spécifique n'a manifestement pas d'autre fonction que de servir de support.

Enfin, on notera que l'effet bénéfique du support principalement composé de silice à haute surface spécifique sur l'activité du catalyseur est étroitement lié à la nature des métaux entrant dans la composition du catalyseur. La demanderesse a observé, en effet, que l'on ne retrouve en aucune manière l'effet bénéfique du support à base de silice à haute surface spécifique lorsqu'on remplace dans le catalyseur soit le nickel par le cobalt, soit encore le palladium par d'autres éléments catalytiques actifs, comme le platine ou le molybdène.

On se propose maintenant de décrire plus en détail le catalyseur et sa préparation, de même que le procédé d'hydrogénation catalytique de l'anhydride maléique selon la présente invention.

1. Catalyseur.

Le catalyseur utilisé dans le procédé de la présente invention contient comme éléments catalytiquement actifs, du nickel et du palladium sous la forme métallique.

Les matières premières utilisées pour la préparation de ce catalyseur sont des composes qui sont solubles dans l'eau et qui peuvent fournir par réduction thermique, les éléments Ni et Pd sous la forme métallique. Des composés de ce genre sont par exemple les suivants:
– nickel: nitrate, formiate, oxalate, tartrate, citrate, etc.;
– palladium: chlorure, complexes aminés tels que le chlorure de palladium (II) tétrammine, [Pd(NH$_3$)$_4$]Cl$_2$, etc.

Conformement à la présente invention, les éléments catalytiquement actifs (nickel et palladium) sont utilisés sur un support à base de silice présentant une surface spécifique d'au moins 100 m²/g (déterminée par la méthode B.E.T./N$_2$; ASTM D 3663-84). Les supports à base de silice préférés ont des surfaces spécifiques d'environ 100 m²/g à 800 m²/g et davantage.

Les supports à base de silice à haute surface spécifique qui peuvent être utilisés conformément à l'invention ont une teneur en SiO$_2$ d'au moins 70% en poids. Les supports préférés contiennent entre 75 et 100% en poids de SiO$_2$.

Lorsque le support à haute surface spécifique n'est pas constitué entièrement de silice, celle-ci est associée à d'autres oxydes inorganiques réfractaires, tels que l'oxyde d'aluminium, le dioxyde de titane, etc.

Ces supports à haute surface spécifique peuvent être obtenus par des méthodes connues en soi, comme par exemple celle décrite par A.J. LEONARD et coll. dans Discussions Faraday Soc.,52,(1971),98-108. A titre d'exemple, une silice à haute surface spécifique peut être obtenue à partir d'orthosilicate de tétraéthyle par hydrolyse au moyen d'une solution d'un acide, tel que l'acide acétique, à une température de 80-90°C et calcination du gel obtenu à des températures comprises entre 400 et 1000°C. On peut également se servir de supports à haute surface spécifique disponibles dans le commerce; elles sont vendues par exemple par les sociétés RHONE-POULENC, DEGUSSA, W.R.GRACE Inc., REDCO N.V.

Les supports utilisés conformément à l'invention se présentent sous la forme de poudres dont la dimension des particules est comprise entre 5 et 100 μm.

Lorsqu'on utilise des supports de composition chimique différente, comme par exemple l'oxyde d'aluminium, l'oxyde de magnésium, le phosphate d'aluminium, les alumino-silicates dont la teneur en SiO$_2$ est faible, les zéolites, on constate une chute nette de l'activité du catalyseur.

Comme on le verra plus loin dans les exemples de réalisation, d'excellents resultats peuvent être obtenus avec des catalyseurs dont la teneur en nickel (sous forme de métal) est seulement de 8 à 25% en poids et dont la teneur en palladium (sous forme de métal) est seulement de 0,5 à 4% en poids par rapport au poids total du catalyseur supporté.

## 2. Préparation du catalyseur.

Les composants métalliques du catalyseur peuvent être déposés sur le support à base de silice selon des méthodes bien connues de l'homme de métier.

Ainsi, par exemple, on peut commencer par imprégner le support pulvérulent avec une solution aqueuse contenant un composé d'un des deux métaux, sécher la pâte ainsi obtenue, imprégner ensuite la poudre sèche avec une solution aqueuse d'un composé de l'autre métal et enfin sécher à nouveau la pâte ainsi obtenue. Mais, on peut tout aussi bien imprégner le support avec une solution aqueuse contenant les deux composés métalliques à la fois, suivi d'un seul séchage de la pâte obtenue. On peut également envisager de précipiter d'abord le composé de nickel sur le support, ensuite le composé de palladium, et vice versa. Il est évident que le dépôt des composants métalliques sur le support peut être effectué dans un ordre quelconque.

Les opérations de séchage peuvent être effectuées en présence d'air, à une température de 80 à 150°C pendant une durée de 3 à 20 heures. Ensuite, on calcine la matière catalytique supportée en présence d'un gaz contenant de l'oxygène, par exemple de l'air, à une température de 300 à 750°C pendant une durée de 0,5 à 3 heures, de préférence à une température de 400 à 500°C pendant une durée de 1 à 2 heures.

Par ce traitement oxydant, les composés de Ni et de Pd contenus dans le support à base de silice sont convertis en oxydes.

Au terme de ce traitement oxydant, on procède à la réduction du catalyseur avec de l'hydrogène pur ou avec un mélange gazeux contenant de l'hydrogène et de l'azote dont la teneur en hydrogène peut varier entre 5 et 100% en volume. Cette réduction est effectuée à une température de 300 à 750°C pendant 0,5 à 5 heures, de préférence à une température de 400 à 550°C pendant 1 à 3 heures.

On refroidit ensuite le catalyseur sous le courant du gaz réducteur jusqu'à une température comprise entre 80 et 150°C. A cette température, on remplace le gaz réducteur par un courant de CO$_2$ pendant 5 à 20 heures. On laisse ensuite refroidir le catalyseur sous courant de CO$_2$ jusqu'à la température ambiante, et l'on remplace progressivement le CO$_2$ par de l'air. Le catalyseur prêt à l'emploi se présente sous la forme d'une poudre en particules de 5 à 300 μm.

## 3. Hydrogénation catalytique de l'anhydride maléique.

Selon la présente invention, l'hydrogénation catalytique de l'anhydride maléique est effectuée en phase liquide avec de l'hydrogène à chaud, sous pression et en présence du catalyseur supporté décrit plus haut. Ce dernier

EP 0 339 012 B1

est introduit dans la phase liquide sous la forme pulvérulente de manière à réaliser une suspension. On sait que l'anhydride maléique est une substance solide à température ordinaire (point de fusion: 52,5°C environ). Pour réaliser l'hydrogénation en présence d'un catalyseur en suspension, il est possible de n'utiliser que de l'anhydride maléique à l'état fondu. Toutefois, cette manière d'opérer présente des difficultés de contrôle de l'exothermicité de la première hydrogénation de l'anhydride maléique en anhydride succinique, avec un risque d'élévation importante de la température. C'est pourquoi, suivant une forme préférée du procédé conforme à l'invention, on exécute l'hydrogénation catalytique de l'anhydride maléique en solution dans un solvant inerte à la réaction ou dans le produit de la réaction.

Le procédé peut être conduit dans un grand nombre de solvants. De préférence, on choisit le solvant parmi les composés suivants utilisés seuls ou en mélange: dioxane, tétrahydrofuranne, gamma-butyrolactone, hydrocarbures aliphatiques, etc.

En général, la concentration de l'anhydride maléique dans le solvant peut varier entre 10 et 90% en poids et de préférence entre 40 et 60% en poids.

En outre, la quantité de catalyseur utilisée dans la mise en oeuvre du procédé conforme à l'invention peut varier entre 1 et 30% en poids par rapport à l'anhydride maléique soumis à l'hydrogénation. Cette quantité est avantageusement comprise entre 5 et 15% en poids par rapport à l'anhydride maléique.

Un avantage essentiel du point de vue technologique du procédé conforme à l'invention est qu'il est exécuté sous des pressions inférieures à 100 bars, c'est-à-dire sous des pressions nettement inférieures à celles appliquées dans l'état de la technique, ce qui autorise l'emploi de réacteurs et instruments conventionnels d'un fonctionnement et d'un entretien commodes et économiques. Dans le mode choisi pour l'exploitation industrielle, la pression opératoire se situe avantageusement entre environ 50 et 100 bars.

Une caractéristique opératoire essentielle du procédé conforme à l'invention est aussi le maintien d'une température d'hydrogénation comprise entre 180 et 270°C, de préférence entre 200 et 240°C. Il est possible d'opérer à une température plus élevée, mais l'on constate que la sélectivité pour la production de gamma-butyrolactone diminue au profit de la formation de sous-produits, tels que l'acide butyrique. Par ailleurs, si l'on opère au-dessous de 180°C, la vitesse de réaction diminue, ce qui nécessite une durée de réaction plus longue et conduit à une diminution de la productivité de l'appareillage de réaction.

Conformément à l'invention, on exécute le procédé d'hydrogénation catalytique de l'anhydride maléique en gamma-butyrolactone par exemple de la manière suivante:

On ajoute dans un réacteur, par exemple un autoclave muni d'un système d'agitation, la quantité désirée d'anhydride maléique, de solvant et de catalyseur, on purge ensuite le réacteur avec de l'azote, puis on remplace l'azote par de l'hydrogène sous une pression de 50 bars et l'on fait fonctionner les moyens d'agitation et de chauffage. Dès que la température atteint 200-240°C, on admet un supplément d'hydrogène pour maintenir constamment la pression réactionnelle à environ 95 bars pendant toute la durée de la réaction.

La durée de la réaction est réglée en sorte d'obtenir plus de 90 moles % de conversion. Suivant les paramètres opératoires choisis, la durée de réaction est comprise entre 1 et 10 heures, de préférence entre 1,5 et 3,5 heures. Au terme de la réaction, on refroidit et détend le contenu de l'autoclave, on sépare par filtration le catalyseur et on récupère le filtrat. Ensuite, on recycle le catalyseur pour effectuer une nouvelle hydrogénation dans les mêmes conditions. Si l'on constate finalement après plusieurs réactions que le catalyseur a subi une perte d'activité, on en remplace une partie par du catalyseur frais pour restaurer son activité. D'autre part, on soumet le filtrat à une séparation, par exemple à une distillation fractionnée pour récupérer la gamma-butyrolactone. Dans le procédé conforme à l'invention, on atteint très facilement des taux de conversion de 90 à 98 moles %; en même temps on obtient des sélectivités très élevées pour la production de gamma-butyrolactone, qui sont toujours supérieures à 93 moles % et atteignent le plus souvent 95-97 moles %. Il ne se forme qu'une très faible quantité de sous-produits (acide propionique, acide butyrique), ce qui facilite grandement les opérations de purification de la gamma-butyrolactone.

Le procédé de l'invention est en outre d'un intérêt économique considérable, étant donné que le catalyseur présente une excellente stabilité et peut être recyclé de nombreuses fois sans aucune diminution de ses performances catalytiques (cf. exemple 6 ci-après).

Les exemples de mise en oeuvre donnés ci-après illustrent l'invention sans la limiter. Dans ces exemples, on utilise les définitions suivantes:

$$- \text{Conversion (en moles \%)} = 100 - \frac{\text{moles d'AS non transformé}}{\text{moles d'AM de départ}} \times 100;$$

$$- \text{Sélectivité en gamma-butyrolactone (en moles \%)} = 100 \times \frac{\text{moles de GBL produite}}{\text{moles d'AM de départ} - \text{moles d'AS non transformé}}.$$

AS = anhydride succinique

6

AM = anhydride maléique

GBL = gamma-butyrolactone.

(En fait, on détermine la conversion de l'anhydride succinique, parce que dans les conditions de la réaction la conversion de l'anhydride maléique est toujours de 100%).

Exemple 1. Préparation du catalyseur.

Cet exemple illustre deux modes de préparation du catalyseur nickel-palladium supporté utilisé selon l'invention.

(a) On dissout 148,6 g de nitrate de nickel ($Ni(NO_3)_2.6H_2O$) dans 185,8 ml d'eau distillée. On imprègne au moyen de cette solution 100 g d'un support de silice présentant une surface spécifique ($BET/N_2$) de 570 $m^2/g$ (préparé selon la méthode décrite par A.J. LEONARD et coll. dans Discussions Faraday Soc.52,(1971),p.98-108, à partir d'orthosilicate de tétraéthyle par hydrolyse au moyen d'une solution d'acide acétique, à une température d'environ 82°C, suivie d'une calcination du gel obtenu à une température de 500°C). On mélange soigneusement le tout et on sèche la pâte ainsi obtenue au four à 100°C pendant 15 heures.

On dissout 7,15 g de $[Pd(NH_3)_4]Cl_2$ dans 185,8 ml d'eau distillée et on imprègne la poudre contenant le sel de nickel et le support au moyen de cette solution. On sèche la pâte obtenue au four à 100°C pendant 15 heures.

On obtient ainsi une poudre que l'on calcine en présence d'air pendant 3 heures à 450°C. On laisse refroidir la poudre ainsi calcinée, puis on procède à la réduction du catalyseur. A cet effet, on remplace l'air par un courant d'hydrogène à un débit de 75 ml/min et l'on porte la température du four à 450°C. On maintient le courant d'hydrogène à cette température pendant une heure.

On refroidit le catalyseur sous courant d'hydrogène jusqu'à atteindre une température de 100°C. A cette température, l'hydrogène est remplacé par un courant de $CO_2$ à un débit de 10 ml/min. On maintient le courant de $CO_2$ pendant 15 heures. Ensuite, on refroidit le catalyseur ainsi obtenu jusqu'à la température ambiante. On remplace alors progressivement le $CO_2$ par de l'air.

Le catalyseur ainsi préparé contient 22,54% en poids de Ni et 2,33% en poids de Pd, calculés par rapport au poids total du catalyseur supporté. Ce catalyseur se présente sous la forme d'une poudre Dans ce qui suit, ce catalyseur est désigné par "catalyseur A".

(b) On dissout 148,6 g de nitrate de nickel ($Ni(NO_3)_2.6H_2O$) dans 145,3 ml d'eau distillée. On dissout par ailleurs 7,15 g de $[Pd(NH_3)_4]Cl_2$ dans 40,5 ml d'eau distillée. On mélange ces deux solutions. On imprègne au moyen de cette solution 100 g du support de silice décrit en (a) ci-dessus et présentant une surface spécifique de 570 $m^2/g$.

On mélange soigneusement le tout et on sèche la pâte ainsi obtenue au four à 100°C pendant 15 heures.

Le traitement ultérieur (calcination, réduction et passivation) est exactement identique à celui décrit pour le catalyseur A.

Le catalyseur ainsi préparé contient les mêmes quantités en poids de nickel et de palladium que le catalyseur A. Ce catalyseur est appelé par la suite "catalyseur B".

Exemple 2. Préparation de la gamma-butyrolactone.

(a) En présence du catalyseur A de l'exemple 1.

Dans un autoclave de 300 ml en acier inoxydable muni d'un système d'agitation magnétique et d'un manteau chauffant électrique, on introduit 56 g d'anhydride maléique, 56 g de tétrahydrofuranne et 5,6 g de catalyseur A préparé à l'exemple 1(a). Après avoir dégazé le contenu du réacteur avec de l'azote, puis avec de l'hydrogène, on porte la pression du réacteur à 50 bars avec de l'hydrogène. Ensuite, on porte la température du mélange progressivement à 235°C, sous agitation, tout en maintenant la pression à 50 bars par un apport d'hydrogène. Lorsque la température de 235°C est atteinte, on élève la pression du réacteur à 95 bars, et l'on maintient cette température constante par le système de régulation du manteau chauffant électrique du réacteur.

Après trois heures de réaction (y compris le temps de chauffe), on refroidit le mélange réactionnel jusqu'à la température ambiante, et on filtre le catalyseur.

L'analyse du mélange réactionnel est effectué par chromatographie en phase gazeuse et par chromatographie en phase liquide sous haute pression. La conversion est de 95 moles % et la sélectivité en gamma-butyrolactone est de 96 moles %.

(b) En présence du catalyseur B de l'exemple 1.

Le procédé est exactement le même qu'en (a) ci-dessus, mais on utilise le catalyseur B préparé à l'exemple 1(b).

D'après l'analyse du mélange réactionnel, la conversion est de 96 moles % et la sélectivité en gamma-butyrolactone de 97 moles %.

Exemple 3. Effet de la surface spécifique du support sur l'activité du catalyseur.

Dans cet exemple, on montre l'incidence de la surface spécifique du support sur l'activité du catalyseur.

A cet effet, on compare les performances catalytiques d'une série de catalyseurs nickel-palladium déposés sur des supports de silice présentant des surfaces spécifiques (BET/N$_2$) allant de 120 à 770 m²/g (conformes à l'invention) avec celles de catalyseurs nickel-palladium déposés sur du kieselguhr (fourni par la société RIEDEL-de HAEN A.G.), dont la surface spécifique (BET/N$_2$) est seulement de 10 m²/g (non conformes à l'invention).

Tous les catalyseurs conformes à l'invention ont été préparés selon le mode opératoire décrit à l'exemple 1(a). Toutefois, pour obtenir des surfaces spécifiques différentes, la calcination du support est effectuée respectivement à 900°C (catalyseur C), 700°C (catalyseur D), 500°C (catalyseur E) et 400°C (catalyseur F).

L'hydrogénation catalytique de l'anhydride maléique est effectuée dans les mêmes conditions qu'à l'exemple 2 (temps de réaction: 3 heures). Le tableau I ci-après indique pour chaque catalyseur mis en oeuvre la quantité en % en poids de nickel et de palladium calculée par rapport au poids total du catalyseur supporté, la nature du support, sa surface spécifique BET/N$_2$ (en m²/g), de même que les conversions et les sélectivités (en moles %) obtenues dans la préparation de la gamma-butyrolactone.

### TABLEAU I.

| Catalyseur | Quantité de métal (% en poids) | | Support | | Conversion (moles %) | Sélectivité (moles %) |
|---|---|---|---|---|---|---|
| | Ni | Pd | Nature | Surface spécifique (m²/g) | | |
| C | 16,26 | 2,06 | SiO$_2$ | 120 | 92 | 94,7 |
| D | 16,26 | 2,06 | SiO$_2$ | 320 | 87 | 93,8 |
| E | 16,26 | 2,06 | SiO$_2$ | 570 | 92 | 95,2 |
| F | 22,5 | 2,33 | SiO$_2$ | 770 | 95 | 93,6 |
| 1 | 16,26 | 2,06 | Kieselguhr | 10 | 62 | - |
| 2 | 22,5 | 0,96 | Kieselguhr | 10 | 60 | - |
| 3 | 22,5 | 1,3 | Kieselguhr | 10 | 62 | - |
| 4 | 22,5 | 2,33 | Kieselguhr | 10 | 61 | - |

Ce tableau montre clairement que l'activité du catalyseur nickel-palladium est augmentée considérablement par l'emploi d'une silice à haute surface spécifique comme support.

On voit en effet que les conversions obtenues (87 à 95 moles %) avec les catalyseurs C à F déposés sur un support de silice à haute surface spécifique (conforme à l'invention) sont nettement supérieures à celles obtenues avec les catalyseurs 1 à 4 déposés sur un support de kieselguhr à faible surface spécifique (60 à 62 moles %) et utilisés comme témoins. A titre comparatif, on a également préparé des catalyseurs déposés sur un support de silice à haute surface spécifique (570 m2/g) selon le mode opératoire décrit à l'exemple 1(a), mais dans lesquels

(1) le palladium a été remplacé par du platine (catalyseur témoin 5) ou du molybdène (catalyseurs témoins 7 et 8),

(2) le nickel a été remplacé par du cobalt (catalyseur témoin 6).

L'hydrogénation catalytique de l'anhydride maléique est effectuée dans les mêmes conditions qu'à l'exemple 2 (temps de réaction: 3 heures). Le tableau II ci-après reproduit les résultats obtenus:

### TABLEAU II.

| Catalyseur | Quantité de métal (% en poids) | | | | | Conversion (moles %) | Sélectivité (moles %) |
|---|---|---|---|---|---|---|---|
| | Ni | Co | Mo | Pd | Pt | | |
| E | 16,26 | - | - | 2,06 | - | 92 | 94,7 |
| 5 | 16,26 | - | - | - | 2,06 | 52,7 | 93,8 |
| 6 | - | 16,26 | - | 2,06 | - | 45 | 95,0 |
| 7 | 16,39 | - | 1,69 | - | - | 61,3 | 94,6 |
| 8 | 50,77 | - | 5,20 | - | - | 62 | 95,2 |

Il apparaît clairement que les catalyseurs 5 à 8 à base de métaux autres que le nickel ou le palladium n'ont qu'une faible activité lorsque le support est une silice à haute surface spécifique. Les conversions (de 45 à 62 moles %) ne dépassent pas celles obtenues avec le catalyseur nickel-palladium déposé sur un support de kieselguhr à faible surface spécifique. L'effet bénéfique de la silice à haute surface sur l'activité catalytique paraît donc étroitement lié à la nature des métaux entrant dans la composition du catalyseur.

Exemple 4. Influence de la quantité de nickel et de palladium.

On utilise pour l'essai une série de catalyseurs préparés exactement comme décrit à l'exemple 1(a) à partir des mêmes matières premières, mais dans des proportions différentes, de manière à faire varier la quantité de métal (nickel et palladium) déposé sur le support de silice à haute surface spécifique (BET/N$_2$) de 570 m$^2$/g; on exécute l'hydrogénation de l'anhydride maléique dans les conditions d'appareillage, de température et de pression de l'exemple 2.

Le tableau III ci-après indique pour chaque catalyseur mis en oeuvre la quantité en % en poids de nickel et de palladium, calculée par rapport au poids total du catalyseur supporté, le temps de réaction requis, de même que les résultats obtenus dans la préparation de la gamma butyrolactone.

### TABLEAU III.

| Catalyseur | Quantité de métal (% en poids) | | Temps de réaction (min) | Conversion (moles %) | Sélectivité (moles %) |
|---|---|---|---|---|---|
| | Ni | Pd | | | |
| G | 22,5 | 0,96 | 300 | 87,4 | 96,4 |
| H | 22,5 | 1,9 | 180 | 92,4 | 95,9 |
| I | 22,5 | 2,33 | 180 | 95,4 | 96,0 |
| J | 8,89 | 2,30 | 330 | 89,6 | 96,0 |

Le tableau III montre qu'il est possible d'obtenir d'excellentes conversions (87 à 96 moles %) et des sélectivités très élevées de 96 moles % en gamma-butyrolactone avec des catalyseurs dont la teneur en nickel (sous forme de métal) varie de 8 à 25% en poids et dont la teneur en palladium (sous forme de métal) varie de 0,8 à 2,5% en poids par rapport au poids total du catalyseur supporté.

Exemple 5. Influence de la température d'hydrogénation.

On opère dans les mêmes conditions qu'à l'exemple 2, mais on fait varier la température et le temps de réaction. Le catalyseur K utilisé dans cet essai est préparé selon le mode opératoire décrit à l'exemple 1(a) mais il contient 16,42% en poids de nickel et 1,04% en poids de palladium, calculés par rapport au poids du catalyseur supporté.

Les résultats obtenus sont repris au tableau IV suivant:

TABLEAU IV.

| Catalyseur | Température (°C) | Temps de réaction (min) | Conversion (moles %) | Sélectivité (moles %) |
|---|---|---|---|---|
| K | 235 | 360 | 92,6 | 96 |
| K | 270 | 120 | 93 | 93 |

On voit donc qu'à température plus élevée (270°C), la sélectivité en gamma-butyrolactone diminue. La perte de sélectivité est due à la production de sous-produits et surtout d'acide butyrique. Ce produit est très difficilement séparé de la gamma-butyrolactone par distillation, vu que son point d'ébullition est très proche de celui de la gamma-butyrolactone.

Exemple 6. Recyclage du catalyseur.

On exécute l'hydrogénation catalytique de l'anhydride maléique dans les mêmes conditions qu'à l'exemple 2 (temps de réaction: 3 heures) en utilisant le catalyseur A préparé selon le mode opératoire décrit à l'exemple 1(a). Toutefois, après séparation du catalyseur par filtration, on le recycle pour effectuer une nouvelle hydrogénation dans les mêmes conditions. On répète cette opération 30 fois.

Le tableau V ci-après reproduit les performances du catalyseur au cours de ces différentes opérations:

TABLEAU V.

| Nombre de recyclages | Conversion (en moles %) | Sélectivité (moles %) |
|---|---|---|
| - | 94,7 | 96 |
| 1 | 95 | 97 |
| 5 | 93,3 | 97,1 |
| 10 | 96,2 | 94,9 |
| 15 | 94,1 | 95,2 |
| 20 | 94,0 | 96,1 |
| 25 | 93,9 | 95,6 |
| 30 | 93,2 | 95,7 |

On voit que le catalyseur peut être recyclé de nombreuses fois sans signe de désactivation appréciable. Après trente recyclages, le catalyseur conserve son activité et sa sélectivité de façon excellente.

Exemple 7. Influence de la composition chimique du support.

Dans cet exemple, on montre l'incidence de la composition chimique du support sur l'activité du catalyseur. On utilise pour l'essai une série de catalyseurs préparés exactement comme décrit à l'exemple 1(a) avec les mêmes quanités de métaux, mais déposés sur des supports dont la composition chimique ou l'origine est différente.

L'hydrogénation catalytique de l'anhydride maléique est effectuée dans les mêmes conditions qu'à l'exemple 2 (temps de réaction: 3 heures). Le Tableau VI ci-après indique pour chaque catalyseur mis en oeuvre la composition chimique du support, sa provenance, sa surface spécifique BET/$N_2$ (en $m^2/g$), de même que les conversions et les sélectivités (en moles %) obtenues dans la préparation de la gamma-butyrolactone.

## TABLEAU VI

| Catalyseur | Support | | | Conversion (moles %) | Sélectivité (moles %) |
|---|---|---|---|---|---|
| | Composition chimique | Origine | Surface spécifique ($m^2/g$) | | |
| L | $SiO_2$ | * | 610 | 95,4 | 95,1 |
| M | $SiO_2$ | W.R. GRACE, Inc. (Silica Gel 239) | 390 | 90 | 97,1 |
| N | $SiO_2/Al_2O_3$ (87/13) | * | 345 | 95 | 95,4 |
| O | $SiO_2/Al_2O_3$ (86,5/13,5) | REDCO N.V. (XONOSIAL) | 440 | 98 | 94,7 |
| 9 | $SiO_2/Al_2O_3$ (50/50) | * | 320 | 85 | 94 |
| 10 | $Al_2O_3$ | * | 230 | 35 | 93 |
| 11 | MgO | * | 310 | 40 | 94,2 |
| 12 | Zéolite (NaY) | UNION CARBIDE | 800 | 51,5 | 92,7 |

\* ces supports ont été préparés selon le mode opératoire indiqué à l'exemple 1(a).

On voit que les conversions obtenues (90 à 98 moles %) avec les catalyseurs L à O déposés sur un support à base de silice (conforme à l'invention), sont nettement supérieures à celles obtenues avec les catalyseurs 9 à 12 déposés sur un support de composition différente (non conforme à l'invention) en dépit du fait que ces derniers ont une surface spécifique supérieure à 200 $m^2/g$.

En outre, on voit que l'on n'obtient une conversion élevée qu'à condition d'utiliser un support à base de silice à forte teneur en $SiO_2$. De plus, il est clair que l'origine des supports à base de silice a peu d'influence sur les résultats.

## Revendications

1. Procédé de fabrication de gamma-butyrolactone par hydrogénation catalytique de l'anhydride maléique en phase liquide avec de l'hydrogène à température élevée et sous pression en présence d'un catalyseur constitué essentiellement de nickel et de palladium déposés sur un support et dont la teneur en nickel est de 8 à 25% en poids et la teneur en palladium est de 0,5 à 4% en poids par rapport au poids total du catalyseur supporté, caractérisé en ce que le support est un support à base de silice présentant une surface spécifique d'au moins 100 m²/g.

2. Procédé selon la revendication 1, caractérisé en ce que la surface spécifique du support à base de silice est comprise entre environ 100 m²/g et environ 800 m²/g.

3. Procédé selon l'une quelconque des revendications 1 et 2, caractérisé en ce que la teneur en SiO₂ du support est comprise entre 70 et 100% en poids, le restant étant constitué d'un autre oxyde inorganique réfractaire.

4. Procédé selon l'une quelconque des revendications 1 à 3, caractérisé en ce que le support est une silice substantiellement pure

5. Procédé selon l'une quelconque des revendications 1 à 4, caractérisé en ce que l'hydrogénation est effectuée à une température comprise entre 180 et 270°C et sous une pression comprise entre environ 50 et 100 bars.

6. Procédé selon l'une quelconque des revendications 1 à 5, caractérisé en ce que l'hydrogénation est effectuée à une température comprise entre 200 et 240°C et sous une pression comprise entre environ 50 et 100 bars.

7. Procédé selon l'une quelconque des revendications 1 à 6, caractérisé en ce que l'hydrogénation est effectuée dans un solvant inerte à la réaction ou dans le produit de la réaction.

8. Procédé selon l'une quelconque des revendications 1 à 7, caractérisé en ce que la quantité de catalyseur utilisée est comprise entre 1 et 30%, de préférence entre 5 et 15% en poids par rapport à l'anhydride maléique soumis à l'hydrogénation.

## Claims

1. Process for producing gamma-butyrolactone by catalytic hydrogenation of maleic anhydride in the liquid phase with hydrogen, at high temperature and under pressure, in the presence of a catalyst consisting essentially of nickel and palladium deposited on a support and wherein the nickel content ranges from 8 to 25% by weight and the palladium content ranges from 0,5 to 4% by weight based on the total weight of the supported catalyst, characterised in that the support is a silica based support having a specific surface area of at least 100 m²/g.

2. Process according to claim 1, characterised in that the specific surface area of the silica based support is between about 100 m²/g and about 800 m²/g.

3. Process according to any of claims 1 and 2, characterised in that the SiO₂ content of the support is between 70 and 100% by weight, the balance consisting of another refractory inorganic oxide.

4. Process according to any of claims 1 to 3, characterised in that the support is a substantially pure silica.

5. Process according to any of claims 1 to 4, characterised in that the hydrogenation is carried out at a temperature between 180 and 270°C and under a pressure of between about 50 and 100 bars.

6. Process according to any of claims 1 to 5, chracterised in that the hydrogenation is carried out at a temperature between 200 and 240°C and under a pressure between about 50 and 100 bars

7. Process according to any of claims 1 to 6, characterised in that the hydrogenation is carried out in a solvent which is inert to the reaction or in the product of the reaction.

8. Process according to any of claims 1 to 7, characterised in that the amount of catalyst used is between 1 and 30%, preferably between 5 and 15% by weight with respect to the maleic anhydride subjected to the hydrogenation.

## Patentansprüche

1. Verfahren zur Herstellung von Gamma-Butyrolacton durch katalytische Hydrierung von Maleinsäureanhydrid in flüssiger Phase mit Wasserstoff bei erhöhter Temperatur und unter Druck in Gegenwart eines Katalysators, der im wesentlichen aus Nickel und Palladium, die auf einem Träger aufgebracht sind, besteht und, dessen Nickelgehalt 8 bis 25 Gew.-% und dessen Palladiumgehalt 0,5 bis 4 Gew.-% beträgt bezogen auf das Gesamtgewicht des Trägerkatalysators, dadurch gekennzeichnet, daß der Träger ein Träger auf der Basis von Kieselerde ist, der eine spezifische Oberfläche von wenigstens 100 m²/g aufweist.

2. Verfahren nach Anspruch 1, dadurch gekennzeichnet, daß die spezifische Oberfläche des Trägers auf der Basis von Kieselerde zwischen etwa 100 m²/g und etwa 800 m²/g liegt.

3. Verfahren nach einem der Ansprüche 1 und 2, dadurch gekennzeichnet, daß der Gehalt an SiO₂ des Trägers zwischen 70 und 100 Gew.-% liegt, wobei der Rest aus einem anderen hitzebeständigen, anorganischen Oxid besteht.

4. Verfahren nach einem der Ansprüche 1 bis 3, dadurch gekennzeichnet, daß der Träger eine im wesentlichen reine Kieselerde ist.

5. Verfahren nach einem der Ansprüche 1 bis 4, dadurch gekennzeichnet, daß die Hydrierung bei einer Temperatur zwischen 180 und 270°C und unter einem Druck zwischen etwa 50 und 100 Bar durchgeführt wird.

6. Verfahren nach einem der Ansprüche 1 bis 5, dadurch gekennzeichnet, daß die Hydrierung bei einer Temperatur zwischen 200 und 240°C und unter einem Druck zwischen etwa 50 und 100 Bar durchgeführt wird.

7. Verfahren nach einem der Ansprüche 1 bis 6, dadurch gekennzeichnet, daß die Hydrierung in einem für die Reaktion inerten Lösungsmittel oder im Reaktionsprodukt durchgeführt wird.

8. Verfahren nach einem der Ansprüche 1 bis 7, dadurch gekennzeichnet, daß die verwendete Katalysatormenge zwischen 1 und 30 Gew.-%, vorzugsweise zwischen 5 und 15 Gew.-%, liegt bezogen auf das der Hydrierung unterworfene Maleinsäureanhydrid.